# EUROPEAN PATENT APPLICATION

(11) **EP 4 725 492 A1**
(43) Date of publication of application: **15.04.2026**
(21) Application number: 24818843.5
(22) Date of filing: 11.03.2024
(51) Int. Cl.: A61K 31/58, A61K 31/506, A61K 33/04, A61K 33/26, A61P 17/14

(54) **PHARMACOLOGICAL COMPOUND FOR FOLLICULAR UNIT STIMULATION**

(30) Priority: 07.06.2023 ES 202330473
(71) Applicant: Natural Folic Sl, 18008 Granada (ES)
(72) Inventor: FERNÁNDEZ SÁNCHEZ, Antero, 18008 Granada (ES)
(74) Representative: Munoz Garcia, Antonio
(86) International application number: PCT/ES2024/070153
(87) International publication number: WO 2024/252037

(57) **Abstract**

The invention consists of a pharmacological compound especially conceived for follicular unit stimulation, involving finasteride, minoxidil, cystine, methionine, astaxanthin, melatonin, biotin, retinol, iron, selenium, collagen, zinc, molybdenum and vitamin D.

## Description

### TECHNICAL FIELD

The present invention refers to a pharmacological compound for follicular unit stimulation whose purpose is to combat poor hair nutrition, both in healthy persons with genetic disorders as well as in patients of various kinds, where the hair is damaged by said disease.

The object of the invention is to provide a product that produces the stimulation of the follicular unit in humans by means of pharmacological and natural resources to be used in seasonal or continuous effluvium, androgenic alopecia, alopecia secondary to poor vascularization, stress and autoimmune processes.

The compound of the invention stimulates the follicular unit centrally from the medulla with its own nutritional needs to the promontory for a correct formation of the external root epithelium.

The invention is based on the study of the capillary needs for its correct nutrition, adapting the correlations between its various components so that its efficacy is complete.

### BACKGROUND OF THE INVENTION

The maintenance of human hair has been at the center of human interest since ancient times, and hair treatments of various kinds have always been chosen in order to beautify and/or maintain it, so that the fight against alopecia has never had an end.

This type of treatment is applied from the outside (where the follicular unit is not located) with poor results in most cases.

Drugs have also been used for another specialty in which a slight improvement in hair has been observed but without lasting clinical resolution.

Until now there was no truly effective and specific treatment against hair loss.

### DISCLOSURE OF THE INVENTION

The capillary nutritional complex disclosed herein solves the above-mentioned problem in a fully satisfactory manner.

For this purpose, and more specifically, the product of the invention is constituted from the following components:

### Finasteride:

Finasteride is a drug approved by the health authorities for androgenic alopecia. It prevents the conversion of testosterone to its more potent form, dihydrotestosterone (DHT), thus markedly reducing plasma levels of this hormone. Its side effects are dose-dependent and reversible when the drug is discontinued. In a 2016 meta-analysis on 17634 people indicates that it is not associated with significant alterations in sexual function.

### Minoxidil:

Peripheral vasodilator that acts by relaxing the vascular smooth muscles, mainly at the arteriolar level. It is used to stimulate hair growth and slow down baldness. The biotransformation of absorbed minoxidil after topical application is not well known. It is believed that approximately 60% of absorbed minoxidil is metabolized to minoxidil glucuronide, mainly in the liver. Oral minoxidil was found to be more effective than minoxidil lotion. During the study, no patient dropped out of treatment, and they had regular use of both options. The reasons why oral minoxidil is more effective than topical minoxidil are not known at this time.

### Cystine:

To better understand its field of action, we must go inside the hair fiber. Hair is composed of a fibrous protein: alpha keratin. These keratins also have a special structure with intermediate filaments. These are aligned in such a way that they create the hair fiber.

A characteristic feature of alpha keratin is its high sulfur value. It finds its origin in the high content of the di-amino acid cystine. In fact, cysteine and methionine are the only naturally occurring sulfur-containing amino acids. The bonds of this can form disulfide bridges, in which the two cysteine molecules eventually form the diaminioacid cystine.

Knowing these cystine values, we now understand that, with an increase in cystine concentration in the hair follicles, an increase in the growth rate and thickness of the hair fibers is obtained directly.

### Methionine:

It is an essential amino acid for hair growth. It is a potent antioxidant and a storehouse of sulfur, a key ingredient to counteract hair disorders. Due to its lipotropic function, it increases blood flow in the hair follicles, promoting greater hair growth.

### Astaxanthin:

Its antioxidant power is 800 times more powerful than coenzyme Q10 and 6000 times more powerful than vitamin C. It is an antioxidant explosion for all cells.

It renews the skin and increases its health, improves the health of connective tissue, intervenes and improves the health of healthy cell membranes after exposure to UV light, helps to maintain an antioxidant balance.

### Melatonin:

Melatonin, that hormone universally known for its power to regulate circadian rhythms, is also involved in hair growth. Until now, at a popular level, it was linked to its ability to facilitate sleep, but science has long been investigating its relationship with hair. And there is good news, because according to the study Melatonin and hair physiology by Dr. Massimo Milani, its antioxidant and anti-inflammatory activities on the skin and its involvement in hair growth have been demonstrated to promote its anagen phase and attenuate the oxidative stress of the hair follicle.

### Biotin:

Biotin improves the infrastructure of keratin, a basic protein that makes up hair, skin and nails, so it can improve hair health, including volume and scalp coverage.

### Retinol:

Vitamin A and its derivatives (retinoids) are important for the development and maintenance of multiple epithelial tissues, including skin, hair and sebaceous glands, as serious adverse effects are observed in vitamin A deficiency or excess.

Increases blood flow to the hair follicles and stimulates the formation of new blood vessels.

### Iron:

It is the mineral responsible for producing hemoglobin, which is the protein that transports oxygen to the rest of the body. One of its benefits is that it allows oxygen to be transported to the hair roots, thus influencing a healthier and stronger hair.

### Selenium:

Selenium, an anti-infective agent, relieves itching and flaking of the scalp and removes dry, flaky particles commonly referred to as dandruff or seborrhea.

### Collagen:

Collagen is an essential ingredient for healthy and strong hair. This substance, which the body generates naturally, tends to disappear with age, making the hair look dull, weak and brittle.

Keeps hair strong and resistant: thanks to its extra supply of amino acids, hair receives the nutrients it needs to be healthy.

Counteracts hair loss: collagen provides nutrients to the root cells, slowing down hair loss.

It helps accelerate growth: by improving circulation in the area, hair grows faster.

In short, the main advantage of using collagen is that it acts by regenerating the hair in depth.

### Zinc:

To understand the link between hair health and zinc, it is first necessary to understand the connection between zinc insufficiency and hair loss. The lack of zinc is, in fact, the main cause of the deterioration of the structure of the protein that makes up the hair follicle. This weakening of the follicle in turn can cause hair deterioration and alopecia.

Zinc has also been shown to play a crucial role in the production of DNA and RNA, being necessary for the division of hair follicle cells and for healthier hair growth.

At the same time, zinc helps to keep hormone levels balanced, which is one of the essential reasons why it is considered so effective in preventing hair loss and graying. Aged and graying hair can be restored to its original color when nourished with zinc-rich diets or supplements formulated with zinc.

### Molybdenum:

Molybdenum is responsible for the metabolism of sulfur amino acids, in particular methionine, essential for keratin synthesis.

Molybdenum is a mineral we need to stay healthy. The body uses molybdenum to process proteins and genetic material such as DNA. Molybdenum also helps to break down drugs and toxic substances that enter the body.

### Vitamin D:

If your body does not have enough vitamin D, accelerated hair loss may occur, which would be related to alopecia.

This is because a deficiency of this nutrient negatively influences the development of the hair growth cycle and is related to the appearance of autoimmune problems, since vitamin D contributes to the immune processes of the scalp being able to distinguish between bacteria and normal tissue.

Without this privilege, an autoimmune reaction against this tissue inevitably occurs, which results in weakness and vulnerability, altering the hair growth cycle, so that hair falls out prematurely and it is much more difficult for it to grow back.

There are also scientific studies that have proven that vitamin D has the particularity of regulating the expression of genes that promote normal hair follicle growth. In addition, it modulates the production of T-lymphocytes, these immune cells serve as guardians against serious and sudden threats in the body.

More specifically, these elements will participate in the following composition:

| | |
|---|---|
| - Finasteride | between 0.8 and 2.5mg. |
| - Minoxidil | between 0.25 and 5mg. |
| - Cystine | between 200 and 300mg. |
| - Methionine | between 50 and 150mg. |
| - Astaxanthin | between 4 and 12mg. |
| - Melatonin | between 0.2 and 1mg. |
| - Biotin | between 5ng and 5mg. |
| - Retinol | between 300 and 800ng. |
| - Iron | between 6 and 14mg. |
| - Selenium | between 10ng and 150mg. |
| - Collagen | between 10 and 40mg. |
| - Zinc | between 5 and 10mg. |
| - Molybdenum | between 5 and 25ng. |
| - Vitamin D | between 50 and 100ng. |

The product thus described will be taken orally, with daily doses, either in liquid or solid form in any format for oral ingestion, and may be obtained in tablets, sugar-coated tablets, pills, hard or soft capsules.

In the case of capsules, these may contain vegetable or animal material on the outside, as well as glycerin for coating.

This results in a highly effective hair nutritional complex for the treatment of hair.

### EXAMPLE OF PRACTICAL EMBODIMENT OF THE INVENTION

As an example, the following components are used to obtain 276.25037 mg of capillary nutritional complex:

| | |
|---|---|
| - Finasteride | 0,8mg. |
| - Minoxidil | 0,25mg. |
| - Cystine | 200mg. |
| - Methionine | 50mg. |
| - Astaxanthin | 4mg. |
| - Melatonin | 0,2mg. |
| - Biotin | 5ng. |
| - Retinol | 300ng. |
| - Iron | 6mg. |
| - Selenium | 10ng. |
| - Collagen | 1010mg. |
| - Zinc | 5mg. |
| - Molybdenum | 5ng. |
| - Vitamin D | 50ng. |

As mentioned above, the compound can be manufactured in liquid form or in tablets, sugar-coated tablets, pills, hard or soft capsules, determining a compound for stimulation of the follicular unit in humans by means of pharmacological and natural resources to be used in seasonal or continuous effluvium, androgenic alopecia, alopecia secondary to poor vascularization, stress and autoimmune processes.

## Claims

1. Pharmacological compound for follicular unit stimulation, **characterized in that** it has the following composition:
| | |
|---|---|
| - Finasteride | between 0.8 and 2.5mg. |
| - Minoxidil | between 0.25 and 5mg. |
| - Cystine | between 200 and 300mg. |
| - Methionine | between 50 and 150mg. |
| - Astaxanthin | between 4 and 12mg. |
| - Melatonin | between 0.2 and 1mg. |
| - Biotin | between 5ng and 5mg. |
| - Retinol | between 300 and 800ng. |
| - Iron | between 6 and 14mg. |
| - Selenium | between 10ng and 150mg. |
| - Collagen | between 10 and 40mg. |
| - Zinc | between 5 and 10mg. |
| - Molybdenum | between 5 and 25ng. |
| - Vitamin D | between 50 and 100ng. |
